# EUROPEAN PATENT SPECIFICATION

(11) **EP 0 521 573 B1**
(45) Date of publication and mention of the grant of the patent: **03.05.1995**
(21) Application number: 92201970.8
(22) Date of filing: 30.06.1992
(51) Int. Cl.: A61F 2/06

(54) **Expandable ring, cylinder or sleeve which can be made non-deformable**
Aufweitbarer Ring, Zylinder oder Manschette, welcher in einen nicht verformbaren Zustand gebracht werden kann
Anneau, cylindre ou manchon expansible pouvant prendre un état non-déformable

(30) Priority: 03.07.1991 NL 9101159
(43) Date of publication of application: 07.01.1993
(73) Proprietor: INDUSTRIAL RESEARCH B.V., NL-7535 PG Enschede (NL)
(72) Inventor: Glastra, Hendrik, NL-7535 PG Enschede (NL)
(74) Representative: Timmers, Cornelis Herman Johannes

(56) References cited:
- EP-A- 0 428 479
- EP-A- 0 452 219
- WO-A-90/01969
- WO-A-91/07927
- US-A- 3 401 689
- US-A- 3 930 496
- US-A- 4 183 102

## Description

The invention relates to a device for locally supporting or strengthening a body vessel comprising a cylindrical body which, when positioned at the desired location, can be extended radially so as to define a hollow cylindrical cavity and then retains its extended shape due to the presence of curable material in said cylindrical cavity.

Such a device, commently called a "stent", is known from EP-A-0 452 219 which describes an inflatable cylinder or sleeve fixed to the end of a catheter and with its interior space connected to two conduits. For use as a permanent stent the sleeve is to be filled with curable material through one of the conduits; this material then cures when it is irradiated with UV radiation. This is, however, proposal because it is only possible to insert a catheter at the desired place in heart- and brain vessels when the catheter has a diameter as small as 2,5-4 mm; otherwise it is impossible to manipulate it into place. Thus the practical use of this stent is limited. This, very small, diameter which is a pre-requisite for universal use of course also restricts the diameter of the conduits through which the polymeric material is to flow to the sleeve at the tip of the catheter: when possible at all it would take quite a lot of time. Even if it would be possible to press polymeric material through so small a diameter there is another, more technological drawback: with the present technologies it is impossible to fabricate by extrusion conduits with the required diameter and properties.

Furthermore the rupture of the connections between the stent and its supply conduits, necessary when the stent is to remain in place, causes problems. When one waits until the material is completely cured then the material which is present in the transition between the sleeve and its supply conduits is also cured so that it is necessary to excert relatively great forces on the intended breaking point. This brings with it the very real risk that the stent is shifted to another position. However, when one breaks the connection earlier, thus before complete curing is effected, then it is inevitable that part of the, poisonous, curing material present in the stent and the connected conduits leaks into the blood stream. This is highly undesirable and can pose a very real threat for the well-being of the patient.

The invention aims to obviate these drawbacks. According to the invention this is achieved in that the cylindrical body is provided around an inflatable dotter device which is to be withdrawn from it after the curing of the material in its extended shape, is closed in itself and filled with curable material prior to the insertion of the device, the dotter device being in the vicinity of the distal end of a light conducting fiber for transmitting UV radiation from an outside source to the curable material of which the curing is started under the influence of UV radiation.

As a result of the measures according to the invention the combination of stent and inflatable dotter device can be made with a very small diameter so that this combination can easily be manipulated into place and is universally applicable, also for treating heart- and brainvessels; there are no cumbersome conduits and in addition to the usual guidewire only the presence of a light conducting fiber for conducting the UV radiation is necessary and this optical fiber can be made with very small dimensions and does not hinder the use of the device.

The withdrawal of the dotter device can be delayed until complete curing of the material is effected, thus ensuring that the stent remains in its intended place.

Preferred embodiments are described in the claims 2 and 3.

The invention is explained with reference to the drawing, in which;

Figures 1a-1d show several stages of the treatment of a body vessel having a constriction of considerable length.

Figure 2 shows an embodiment according to the invention.

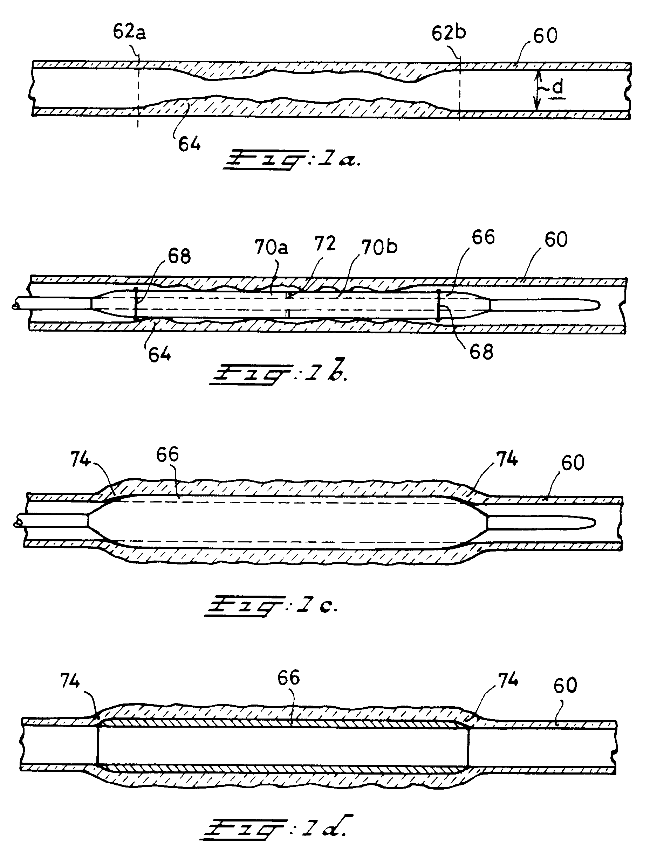

Figure 1a shows how a body vessel 60, particularly a blood vessel has, between the points 62a and 62b a number of constrictions 64 which considerable affect the flow of body fluid. By means of a body scan the distance between the location 62a and 62b is determined and then a device according to the invention is chosen with such a length that it covers said distance. Furthermore the free diameter d of the body vessel is also determined and a stent is chosen with such a diameter that, when it is brought into position and the dotter balloon is inflated it presses itself into the body vessel in such a way that after the curing of its components a smooth transition between the unrestricted part and the stand is obtained.

Figure 1b shows the dotter device 64 and the stent indicated with 66, around it and fixed to the dotter device with the surgical tread 68. In this shown embodiment the stent 66 is here divided into two compartments 70a, 70b separated by the partition 72. It is observed that this embodiment as shown in figs 1a-1d is not the claimed embodiment and that these figures only serve to elucidate how such a cylindrical stent supports a body vessel. This partition can be broken before the dotter - like device is inserted into the body vessel or can be of the kind with breaks when the dotter device 64 is infated. The inflation of the dotter - like device results into the situation according to figure 1c: the stent 66 is pressed into the wall of the vessel 60 to such an extend that there are smooth transitions 74 between the wall of the unrestricted parts and the inner diameter of the stent 66.

When, after curing of the component materials in the stent 66 the dotter device is retracted the stent 66 remains in the body vessel 60 as shown in figure 1d.

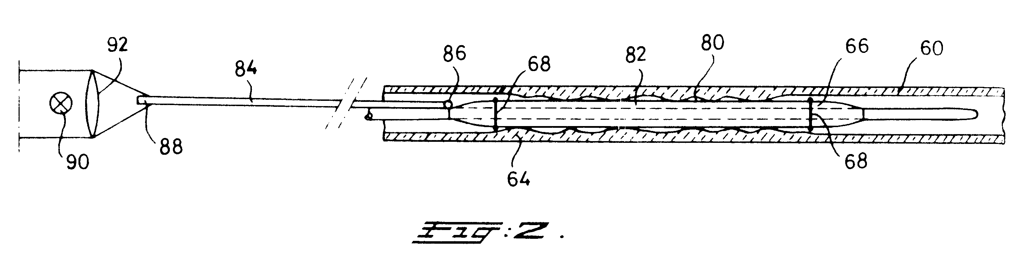

Figure 2 shows the use of a stent 80 according to the invention having only one compartment 82 which is filled with mixture of materials of which the curing starts when it is irradiated with radiation of a suitable wave length, particularly UV light. In the manner as described above this stent is fixed by means of the surgical threads 68 to the dotter - like device 66. Together with the dotter - like device 66 a light conducting glas fiber 84 with at its one end a small lens 86 is inserted into the body; when the whole has reached the desired position the other end 88 is irradiated with ultra violet light from a suitable source 90 via a schematically shown focussing system 92. In this way a considerable amount of UV energy can be transmitted to the desired position, resulting into a rapid curing of the curable material.

## Claims

1. Device (80) for locally supporting or strenghtening a body vessel comprising a cylindrical body (82) which, when positioned at the desired location, can be extended radially so as to define a hollow cylindrical cavity and then retains its extended shape due to the presence of curable material in said cylindrical cavity, **characterised in that** the cylindrical body (82) is provided around an inflatable dotter device (66) which is to be withdrawn from it after the curing of the material in its extended shape, is closed in itself and filled with curable material prior to the insertion of the device, the dotter device (66) being in the vicinity of the distal end (86) of a light conducting fiber (84) for transmitting UV radiation from an outside source (90) to the curable material of which the curing is started under the influence of UV radiation.

2. Device according to claim 1, **characterised in that** the cylindrical body (82) is spirally wound around the uninflated dotter device.

3. Device according to claims 1-2, **characterised in that** the cylindrical body is fixed to the dotter - like device by means of threads (68) which break when the dotter - like device is inflated.

## Patentansprüche

1. Vorrichtung (80) zum örtlichen Abstützen oder Verstärken eines Körpergefäßes, umfassend einen zylindrischen Körper (82), der, wenn er an der gewünschten Stelle angeordnet ist, radial so ausgedehnt werden kann, daß er eine hohle zylindrische Kavität begrenzt, und dann aufgrund des Vorhandenseins von aushärtbarem Material in der zylindrischen Kavität seine ausgedehnte Form beibehält, dadurch gekennzeichnet, daß der zylindrische Körper (82) um eine aufpumpbare Punktiervorrichtung (66) herum angeordnet ist, die nach dem Aushärten des Materials vom zylindrischen Körper in seiner ausgedehnten Form entfernbar ist, daß er weiterhin in sich geschlossen ist und vor dem Einsetzen der Vorrichtung mit härtbaren Material gefüllt worden ist, und daß sich die Punktiervorrichtung (66) in der Nähe des distalen Endes (86) einer Lichtleitfaser (84) zum Übertragen von ultravioletter Strahlung von einer außerhalb befindlichen Quelle (90) zum aushärtbaren Material, dessen Aushärtung unter dem Einfluß der ultravioletten Strahlung begonnen wird, befindet.

2. Vorrichtung nach Anspruch 1, dadurch gekennzeichnet, daß der zylindrische Körper (82) spiralig um die nicht aufgepumpte Punktiervorrichtung gewickelt ist.

3. Vorrichtung nach den Ansprüchen 1 bis 2, dadurch gekennzeichnet, daß der zylindrische Körper an der punktiergerätartigen Vorrichtung mit Hilfe von Fäden (68) befestigt ist, die reißen, wenn die punktiergerätartige Vorrichtung aufgepumpt wird.

## Revendications

1. Dispositif (80) pour supporter ou renforcer localement un vaisseau du corps, comprenant un élément cylindrique (82) qui peut être dilaté radialement, lorsqu'il est placé à la position désirée, de façon à définir une cavité cylindrique creuse, et qui conserve ensuite sa configuration dilatée, du fait de la présence d'une matière durcissable dans ladite cavité cylindrique, caractérisé en ce que l' élément cylindrique (82) est prévu autour d'un dispositif de positionnement gonflable (66) qui doit être retiré dudit élément après durcissement de la matière dans sa configuration dilatée, et ledit élément est fermé en lui-même et rempli de matière durcissable, avant l'insertion du dispositif, le dispositif de positionnement (66) étant situé au voisinage de l'extrémité distale (86) d'une fibre de guidage de lumière (84) pour transmettre un rayonnement ultraviolet d'une source extérieure (90) à la matière durcissable dont le durcissement est déclenché sous l'influence du rayonnement ultraviolet.

2. Dispositif suivant la revendication 1, caractérisé en ce que l'élément cylindrique (82) est enroulé hélicoïdalement autour du dispositif de positionnement non dilaté.

3. Dispositif suivant les revendications 1 et 2, caractérisé en ce que le corps cylindrique est fixé au dispositif de positionnement par des fils (68) qui se cassent lorsqu'on gonfle le dispositif de positionnement.
